(19) Europäisches Patentamt
European Patent Office
Office européen des brevets

(11) Veröffentlichungsnummer: **0 004 639**
**B1**

(12) # EUROPÄISCHE PATENTSCHRIFT

(45) Veröffentlichungstag der Patentschrift: **10.02.82**

(51) Int. Cl.³: **C 07 H 19/20**

(21) Anmeldenummer: **79100940.0**

(22) Anmeldetag: **29.03.79**

(54) **Stabilisierte Nicotinamid-adenin-dinucleotide, Verfahren zu ihrer Herstellung und Verwendung eines Stabilisators.**

(30) Priorität: **01.04.78 DE 2814154**

(43) Veröffentlichungstag der Anmeldung: **17.10.79 Patentblatt 79/21**

(45) Bekanntmachung des Hinweises auf die Patenterteilung: **10.02.82 Patentblatt 82/6**

(84) Benannte Vertragsstaaten: **BE CH DE FR GB IT LU NL SE**

(56) Entgegenhaltungen:
**DE-A-2 740 957**
**DE-B-1 118 792**
**DE-B-1 155 134**

(73) Patentinhaber: **BEHRINGWERKE AKTIENGESELLSCHAFT, Postfach 1140, D-3550 Marburg/Lahn (DE)**

(72) Erfinder: **Stärk, Joseph, Dr., Geschwister-Scholl-Strasse 23, D-3550 Marburg/Lahn (DE)**
Erfinder: **Müller-Matthesius, Reinhard, Dr., Am Weinberg 6c, D-3550 Marburg/Lahn (DE)**

(74) Vertreter: **Meyer-Dulheuer, Karl-Hermann, Dr. et al, HOECHST Aktiengesellschaft Zentrale Patentabteilung Postfach 80 03 20, D-6230 Frankfurt/Main 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG.

# Stabilisierte Nicotinamid-adenin-dinucleotide, Verfahren zu ihrer Herstellung und Verwendung eines Stabilisators

Die Erfindung betrifft stabilisierte Nicotinamid-adenin-dinucleotide, insbesondere das freie Nicotinamid-adenin-dinucleotid in reduzierter oder oxidierter Form und dessen Phosphat, ebenfalls in reduzierter oder oxidierter Form.

In der klinischen Chemie, Biochemie, Lebensmittelchemie, Pharmazie und auf anderen Gebieten werden vielfach enzymatische Reaktionen zu analytischen Zwecken eingesetzt. Insbesondere in der klinisch-chemischen Routinediagnostik werden täglich Metabolite und Enzyme in vielen Blut-, Serum-, Plasma-, Liquor- und Urinproben enzymatisch untersucht. Wegen der Instabilität vieler Komponenten der Reaktion ist es gerade in den klinisch-chemischen Routinelabors üblich geworden, industriell hergestellte Kombinationspackungen zu verwenden, die die Herstellung gebrauchsfertiger Reagenzlösungen mit einem Minimum an Aufwand erlauben.

Es stellt sich deshalb die Aufgabe, Präparate mit möglichst grossem Verwendungszeitraum zu schaffen. Im Interesse einer einfachen Arbeitsweise bei der Verwendung der Reagenzien ist man bestrebt oder gezwungen, möglichst viele der für die Testdurchführung benötigten Stoffe in einen Behälter abzufüllen. Es hat sich jedoch gezeigt, dass verschiedene Substanzen sich in ihrer Stabilität gegenseitig negativ beeinflussen können, auch wenn sie in gefriergetrockneter oder trocken abgefüllter Form vorliegen. Es ist daher von grosser Wichtigkeit, geeignete Zusatzstoffe (Stabilisatoren) aufzufinden, die den Verwendungszeitraum der Reagenzien erhöhen.

Besonders instabile Biochemikalien, die nicht nur in gelöster Form, sondern auch in gefriergetrocknetem oder trocken abgefülltem Zustand bereits für sich grosse Probleme aufwerfen, sind Nicotinamid-adenin-dinucleotid bzw. Nicotinamid-adenin-dinucleotid-phosphat, beide sowohl in ihrer reduzierten wie oxidierten Form. Diese Substanzen sind bei sehr vielen enzymatischen Bestimmungen als Coenzyme notwendig und stellen in der reduzierten Form zugleich den Farbstoff für die photometrische Messung dar.

Als Folge des Zerfalls der genannten Coenzyme kann die nachzuweisende Substanz nicht mehr einwandfrei oder überhaupt nicht mehr fotometriert werden, da der absorbierende Stoff in zu geringer Konzentration vorliegt oder die Geschwindigkeit der zu messenden Reaktion infolge der verminderten Konzentration des Coenzyms zu gering ist. Im Falle der Ermittlung der Aktivität der Lactat-Dehydrogenase (LDH) nach dem Reaktionsschema

$$\text{Pyruvat} + \text{NADH}_2 \xrightarrow{\text{LDH}} \text{L-Lactat} + \text{NAD}$$

kommt als weitere Komplikation hinzu, dass das reduzierte Coenzym Nicotinamid-adenin-dinucleotid ($NADH_2$) im Laufe der Lagerung LDH-Inhibitoren bildet, die zu erheblich falsch-negativen LDH-Werten führen können. Diese Inhibitoren können schon in starkem Masse wirksam werden, bevor ein $NADH_2$-Konzentrationsverlust überhaupt nachweisbar ist.

Aber auch bei vielen anderen wichtigen Bestimmungen wie dem Nachweis von Aspartat-Transaminase, Alanin-Transaminase, $\alpha$-Hydroxybutyrat-Dehydrogenase, Creatin-Kinase, Glutamat-Dehydrogenase, bei der vollenzymatischen Harnstoff-Bestimmung, der enzymatischen Triglycerid-Bestimmung oder bei der Glucose-Bestimmung nach dem Hexokinase-Verfahren gehören Nicotinamid-adenin-dinucleotid bzw. Nicotinamid-adenin-dinucleotid-phosphat in reduzierter bzw. oxidierter Form zu den empfindlichen, den Verwendungszeitraum der Testpackung limitierenden Komponenten, an deren Stabilisierung ein ausserordentliches Interesse besteht.

Aus den deutschen Patentschriften 1 289 668 und 1 598 157 ist Glutathion sowie Cystein, Cystein-Carbamid und Acetyl-Cystein als Stabilisierungsmittel für reduzierte Nicotin-amid-adenin-dinucleotide bekannt. In der Patentschrift 1 930 059 wird Polyvinylpyrrolidon (PVP) als Stabilisator für reduzierte und oxidierte Nicotinamid-adenin-dinucleotid-phosphat beschrieben.

Auch Acacia bzw. Gummi arabicum und Mannit wurden hierfür als geeignet angesehen.

Albumin und Gelatine sind ebenfalls schon als Füllstoffe zu stabilisierten Coenzymen zugesetzt worden (DE 1 930 059).

In der deutschen Offenlegungsschrift 2 740 957 ist ein Verfahren zur Stabilisierung von Nicotinamid-adenin-dinucleotid in flüssiger Phase mit Gelatine beschrieben.

Ein Verfahren zur Stabilisierung biologisch aktiven Materials wie Bakterien, Bakterienstoffwechselprodukten, Viren, Sera und Enzymen ist in der deutschen Patentschrift 1 183 629 beschrieben. Dazu wird eine mit einem Diisocyanat vernetzte hydrolytisch abgebaute Gelatine, vorzugsweise in Kombination mit Natrium-L-Glutaminat verwendet. Wie insbesondere die Beispiele in dieser Patentschrift zeigen, ist ein derartiges Stabilisierungsmittel insbesondere für die Herstellung von Impfstoffen und für die Erhaltung antigener und immunogener Strukturen von Bedeutung.

Die Vielzahl der eingesetzten Stabilisatoren zeigt, dass ihre Wirksamkeit im Hinblick auf Nucleotide noch nicht zufriedenstellend ist.

Überraschend wurde nun gefunden, dass oxidierte und reduzierte Nicotinamid-adenin-dinucleotide und auch deren Phosphate eine bessere Stabilität in bisher bekannten Reagenzien aufweisen, wenn sie eine vernetzte Gelatine enthalten.

Gegenstand der Erfindung ist demnach ein stabiles Gemisch aus

a) Nicotinamid-adenin-dinucleotid in reduzierter oder oxydierter Form oder Nicotinamid-adenin-dinucleotidphosphat in reduzierter oder oxydierter Form und

b) vernetzter Gelatine.

Gegenstand der Erfindung ist ferner ein Verfahren zur Stabilisierung von Nicotinamid-adenin-dinucleotid in reduzierter oder oxidierter Form oder bzw. und von Nicotinamid-adenin-dinucleotid-phosphat in reduzierter oder oxidierter Form, dadurch gekennzeichnet, dass die Lösung des Nucleotids mit einer vernetzten Gelatine versetzt wird, wonach das Gemisch gewünschtenfalls getrocknet, vorzugsweise gefriergetrocknet wird.

Die Konzentration des Gelatineproduktes in der wässrigen Lösung sowie im Trockenprodukt beträgt auf 1 g Nucleotid 0,001 bis 1200 g, vorzugsweise 0,01 bis 300 g, vor allem 0,5 bis 20 g.

Besonders geeignete Gelatineprodukte sind die aus DE 1 118 792 und 1 155 134 bekannten. Sie werden hergestellt, indem man Kollagen oder dessen Abbauprodukte in wässriger Lösung bei einer Temperatur von 60 bis 150 °C bis zu einem Molekulargewicht von 2000 bis 20 000, vorzugsweise 5000 bis 10 000, abbaut, das abgebaute Kollagen mit einem Diisocyanat bei Temperaturen von 0 bis 100 °C im neutralen bis schwach alkalischen pH-Bereich, gegebenenfalls in Gegenwart inerter organischer Lösungsmittel in der Weise umsetzt, dass die angewandte Isocyanatmenge geringer ist als die, welche sich aus der Anzahl der in abgebauten Kollagen vorhandenen Amino- und Guanidinogruppen errechnet, und vorzugsweise etwa 20 bis 80% dieser Menge beträgt, dann das entstandene Vernetzungsprodukt auf einen pH-Wert von etwa 7 einstellt oder dass man Kollagenabbauprodukte mit Diisocyanaten bei Temperaturen von 0 bis 100 °C im neutralen bis schwach alkalischen pH-Bereich, gegebenenfalls in Gegenwart inerter organischer Lösungsmittel, umsetzt, wobei die angewandte Isocyanatmenge 20 bis 80% derjenigen beträgt, die sich aus der Anzahl der vorhandenen Amino- und Guanidinogruppen errechnet, die entstandenen Vernetzungsprodukte in wässriger Lösung bei Temperaturen von 60 bis 150 °C bis zu einem Molekulargewicht von 10 000 bis 100 000 abbaut, die erhaltenen Lösungen auf einen pH-Wert von 7 einstellt. Als 3,5%ige Lösung bei einem mittleren Molekulargewicht von 35 000 sind sie auch als Infusionslösung zur Plasmasubstitution im Handel.

Obwohl der pH-Wert der stabilisierten Nucleotid-Lösung keine entscheidende Bedeutung für den Stabilisierungseffekt hat, empfiehlt es sich, mit Rücksicht auf die empfindlichen Substanzen extreme pH-Werte zu vermeiden. Der bevorzugte pH-Bereich der Lösung liegt demgemäss zwischen pH 5 und 11. Besonders gute Stabilisierungseffekte wurden bei pH-Werten zwischen 7 und 9 beobachtet.

Zur Einstellung des pH-Wertes können die gebräuchlichen Puffersubstanzen verwendet werden. Beispiele für geeignete Puffersubstanzen sind Gemische verschiedener Alkalisalze von schwachen oder mittelstarken Säuren wie Phosphorsäure oder Borsäure, Gemische von schwachen oder mittelstarken Säuren mit ihren Alkalisalzen (z. B. Phthalsäure, Alkaliphthalat) oder Puffer auf Basis von bestimmten organischen Verbindungen wie Tris-hydroxymethylaminomethan oder dergleichen, wie sie allgemein bei biochemischen Reaktionen verwendet werden.

Das nach dem Verfahren erhaltene Gemisch zeigt seine vorzügliche Haltbarkeit insbesondere in getrockneter, vorzugsweise lyophilisierter Form. Das Gewichtsverhältnis des Gelatineproduktes zu Nucleotid im Trockenprodukt entspricht dem der wässrigen Lösung.

Auf diese Weise stabilisiertes Nikotinamid-adenin-dinucleotid weist gegenüber solchem, das mit Gelatine stabilisiert ist, folgende Vorteile auf: Die Lyophilisate lösen sich schneller und lösen sich auch noch, wenn sie mehrere Wochen bei 37 °C gelagert werden, während mit Gelatine stabilisierte, unter diesen Bedingungen gelagerte Lyophilisate sich nur noch unvollständig lösen. Daneben bietet die Verwendung von vernetzter Gelatine produktionstechnische Vorteile: Das Dinucleotid wird bei etwa 2 bis 4 °C verarbeitet. Bei diesen Temperaturen lassen sich bis zu 10%ige Lösungen von vernetzter Gelatine herstellen, während Gelatine unter diesen Bedingungen aus Lösungen mit Konzentrationen über etwa 0,1% auskristallisiert. Ausserdem muss zur Lösung von Gelatine erwärmt werden, um eine genügend hohe Lösungsgeschwindigkeit zu erreichen.

Tabelle

Vergleich der Stabilisierung von $NADH_2$ durch vernetzte Gelatine und Gelatine in Lyophilisaten bei Temperaturbelastung (+37 °C)

| | vernetzte Gelatine $\mu$mol/l $NADH_2$ | Gelatine $\mu$mol/l $NADH_2$ |
|---|---|---|
| Ausgangswert | 200 | 201 |
| 1 Woche | 204 | 206 |
| 2 Wochen | 202 | 199 |
| 3 Wochen | 201 | 199* |
| 4 Wochen | 202 | 200* |

* Lyophilisat partiell unlöslich.

Gegenstand der Erfindung ist ferner die Verwendung einer vernetzten Gelatine zur Stabilisierung von Nicotinamid-adenin-dinucleotid oder bzw. und Nicotinamid-adenin-dinucleotid-phosphat jeweils sowohl in reduzierter als auch in oxidierter Form.

Die erfindungsgemäss stabilisierten trockenen Nicotinamid-adenin-dinucleotide bzw. ihre Mischungen mit dem Gelatineprodukt sind in Wasser leicht löslich. Der erfindungsgemässe Stabilisator besitzt bei den Wellenlängen Hg 334, Hg 366 oder 340 nm eine sehr geringe Eigenextinktion, so dass er sich nicht störend durch Anhebung der Hintergrundextinktion bei der photometrischen Messung bemerkbar macht. Er ist ohne Einfluss auf die Aktivität der üblicherweise als Reagenz verwendeten oder nachzuweisenden Enzyme, z. B. von Alanin-Transaminase, Aspartat-Transaminase, Lactat-Dehydrogenase, Glutamat-Dehydrogenase, Malat-Dehydrogenase, Urease.

Die erfindungsgemäss stabilisierten Nicotinamid-adenin-dinucleotide können entweder für sich oder zusammen mit anderen Komponenten lyophilisiert werden, die für die Testdurchführung notwendig sind. Beispiele für solche Komponenten sind Substrate (z.B. Alanin, Aspartat, Glutamat, Pyruvat, 2-Oxobutyrat, 2-Oxoglutarat), Hilfsenzyme (z.B. Malat-Dehydrogenase, Lactat-Dehydrogenase, Urease, Glutamat-Dehydrogenase), andere Coenzyme wie Adenosindiphosphat oder Adenosintriphosphat oder in der Enzymologie übliche Puffersubstanzen wie Trishydroximethylaminomethan – HCl und andere wie oben angeführt. Die besonders günstige stabilisierende Wirkung des Gelatineproduktes auf Nicotinamid-adenin-dinucleotide geht aus folgenden Beispielen hervor. Hierzu ist zu bemerken, dass die Stabilitätsprüfungen unter drastischer Temperaturbelastung bei + 37 °C durchgeführt werden, um die stabilisierende Wirkung schneller zu erkennen. Normalerweise werden die Präparate jedoch bei +2 bis +6 °C gelagert, wobei wesentlich grössere Stabilitäten resultieren.

Beispiel 1

In Lösungen mit 1,56% des nach DE 1 118 792 erhaltenen Stabilisators oder 2,1% Polyvinylpyrrolidon (PVP), wurde jeweils 1,14 µmol/1 reduziertes Nicotinamid-adenin-dinucleotid ($NADH_2$) gelöst. Das resultierende Gemisch wurde zu gleichen Portionen in Glasflaschen abgefüllt und gefriergetrocknet. Die Glasflaschen wurden bei +37 °C gelagert. Zu verschiedenen Zeitpunkten wurden Proben des Lyophilisats zur Bestimmung der Lactat-Dehydrogenase eingesetzt bzw. die im Lyophilisat verbliebene $NADH_2$-Menge ermittelt.

Die Bestimmung der LDH-Aktivität erfolgte gemäss den Empfehlungen der Deutschen Gesellschaft für Klinische Chemie, Z. Klin. Chem. Klin. Biochem. 10, 182 (1972).

Während der Lagerung des Lyophilisates gebildete Inhibitoren würden sich bei der Bestimmung der LDH-Aktivität durch ein Absinken der Werte bemerkbar machen.

Die im Lyophilisat verbliebene $NADH_2$-Menge wurde nach W. Gerhardt et al., Scand. J. Clin. Lab. Invest. 33, 1 (1974) ermittelt.

| Lagerung bei +37 °C | LDH-Aktivität bei erfindungsgem. Stabilisator | PVP als Stabilisator | $NADH_2$-Menge bei erfindungsgem. Stabilisator | PVP als Stabilisator |
|---|---|---|---|---|
| Ausgangswert | 100% | 100% | 100% | 100% |
| 3 Tage | 98% | 88% | 100% | 94% |
| 7 Tage | 100% | 81% | 100% | 95% |
| 14 Tage | 96% | 65% | 100% | 89% |
| 21 Tage | 100% | 56% | 98% | 82% |
| 28 Tage | 100% | 45% | 99% | 72% |

Beispiel 2

(Harnstoff-Reagenz)

Eine Lösung mit 40 g/l des nach DE 1 155 134 erhaltenen Gelatineproduktes 4,54 µmol/1 $NADH_2$, 357 µmol/1 2-Oxoglutarat und 18 U/ml Glutamat-Dehydrogenase wurde in Glasflaschen gefriergetrocknet. Die Glasflaschen wurden bei +37 °C gelagert.

Zu bestimmten, in der Tabelle wiedergegebenen Zeitpunkten wurden Proben des Lyophilisats in Tris-Puffer, pH 8,0, aufgelöst, mit einem Überschuss von Ammoniumionen versetzt und einer Bestimmung des $NADH_2$-Gehalts auf photometrischem Wege bei 340 nm unterworfen. Das Ergebnis zeigt folgende Tabelle:

| Lagerung bei 37 °C | $NADH_2$-Konzentration |
|---|---|
| Ausgangswert | 100% |
| 14 Tage | 93% |
| 28 Tage | 91% |
| 54 Tage | 88% |
| 84 Tage | 87% |

In gleicher Weise können andere Nucleotid-haltige Reagenzien stabilisiert werden. Ähnlich gute Ergebnisse werden erhalten, wenn man 1–3 g Nucleotid mit 2 bis 20 g Gelatineprodukt versetzt.

Beispiel 3

In jeweils 55 ml Wasser wurden 0,86 g des nach DE 1 118 792 erhaltenen Stabilisators bzw. 0,86 g Polyvinylpyrolidon (PVP) gelöst. In beiden Flüssigkeiten wurden sodann jeweils 54,6 mg reduziertes Nicotinamid-adenin-dinucleatid-phosphat ($NADPH_2$) gelöst, das resultierende Gemisch zu gleichen Portionen in Glasflaschen abgefüllt und gefriergetrocknet. Die Glasflaschen wurden bei +37 °C gelagert. Zu bestimmten, in der Tabelle wiedergegebenen Zeitpunkten wurden die Flaschen geöffnet und der $NADPH_2$-Gehalt des lyophilisierten Inhalts mittels der Reaktion.

$$\text{2-Oxoglutarat} + NADPH_2 + NH_4^{(+)} \xrightarrow{GlDH} \text{Glutamat} + NADP$$

photometrisch gemessen.

Ergebnisse

| Lagerung bei +37 °C | NADPH$_2$-Gehalt erfindungsgemässer Stabilisator | PVP als Stabilisator |
|---|---|---|
| Ausgangswert | 100% | 100% |
| 7 Tage | 100% | 98% |
| 15 Tage | 98% | 93% |
| 21 Tage | 97% | 90% |
| 28 Tage | 94% | 80% |

**Patentansprüche**

1. Stabilisiertes Gemisch aus
a) Nicotinamid-adenin-dinucleotid in reduzierter oder oxydierter Form oder Nicotinamid-adenin-dinucleoitid-phosphat in reduzierter oder oxydierter Form und
b) vernetzter Gelatine.

2. Gemisch nach Anspruch 1, gekennzeichnet durch ein Verhältnis von Nucleotid:vernetzter Gelatine von 1:0,001 bis 1:1200.

3. Verwendung von vernetzter Gelatine als Stabilisator für Nicotinamid-adenin-dinucleotid in reduzierter oder oxydierter Form oder für Nicotinamid-adenin-dinucleotid-phosphat in reduzierter oder oxydierter Form.

4. Verfahren zur Stabilisierung von Nicotinamid-adenin-dinucleotid in reduzierter oder oxydierter Form oder von Nicotinamid-adenin-dinucleotid-phosphat in reduzierter oder oxydierter Form, dadurch gekennzeichnet, dass eine Lösung des Nucleotids mit einer vernetzten Gelatine versetzt und gewünschtenfalls getrocknet wird.

5. Verfahren nach Anspruch 4, dadurch gekennzeichnet, dass eine vernetzte Gelatine verwendet wird, die hergestellt wird, indem man Kollagen oder dessen Abbauprodukte in wässriger Lösung bei einer Temperatur von 60 bis 150 °C bis zu einem Molekulargewicht von 2000 bis 20 000, vorzugsweise 5000 bis 10 000, abbaut, das abgebaute Kollagen mit einem Diisocyanat bei Temperaturen von 0 bis 100 °C im neutralen bis schwach alkalischen pH-Bereich, gegebenenfalls in Gegenwart inerter organischer Lösungsmittel in der Weise umsetzt, dass die angewandte Isocyanatmenge geringer als die, welche sich aus der Anzahl der im abgebauten Kollagen vorhandenen Amino- und Guanidinogruppen errechnet und vorzugsweise etwa 20 bis 80% dieser Menge beträgt, und das entstandene Vernetzungsprodukt auf einen pH-Wert von etwa 7 einstellt oder dass man Kollagenabbauprodukte mit Diisocyanaten bei Temperaturen von 0 bis 100 °C im neutralen bis schwach alkalischen pH-Bereich, gegebenenfalls in Gegenwart inerter organischer Lösungsmittel, umsetzt, wobei die angewandte Isocyanatmenge 20 bis 80% derjenigen beträgt, die sich aus der Anzahl der vorhandenen Amino- und Guanidinogruppen errechnet, die entstandenen Vernetzungsprodukte in wässriger Lösung bei Temperaturen von 60 bis 150 °C bis zu einem Molekulargewicht von 10 000 bis 100 000 abbaut und die erhaltenen Lösungen auf einen pH-Wert von 7 einstellt.

**Claims**

1. Stable mixture of
a) nicotinamide-adenine-dinucleotide in the reduced or oxidized form or nicotinamide-adenine-dinucleotide phosphate in the reduced or oxidized form and
b) cross-linked gelatin.

2. Mixture as claimed in claim 1, wherein the ratio of nucleotide to cross-linked gelatin is in the range of from 1:0.001 to 1:1200.

3. Use of cross-linked gelatin as stabilizer for nicotinamide-adenine-dinucleotide in the reduced or oxidized form or for nicotinamide-adenine-dinucleotide phosphate in the reduced or oxidized form.

4. Process for the stabilization of nicotinamide-adenine-dinucleotide in the reduced or oxidized form or of nicotinamide-adenine-dinucleotide phosphate in the reduced or oxidized form, which comprises adding a cross-linked gelatin to a solution of the nucleotide and optionally drying the same.

5. A process as claimed in claim 4, which comprises using a cross-linked gelatin produced by decomposing collagen or its decomposition products in an aqueous solution at a temperature of from 60 to 150 °C up to a molecular weight of from 2000 to 20.000, preferably from 5000 to 10.000, reacting the decomposed collagen with a diisocyanate at a temperature of from 0 to 100 °C in the neutral to slightly alkaline pH range, optionally in the presence of inert organic solvents in a manner that the amount of isocyanate employed is smaller than that which is calculated from the number of amino and guanidino groups present in the decomposed collagen, and is preferably from about 20 to 80% of this amount, and by subsequently adjusting the cross-linked product obtained to a pH value of about 7; or by reacting decomposition products of collagen with a diisocyanate at a temperature of from 0 to 100 °C in the neutral to slightly alkaline pH range, optionally in the presence of inert organic solvents, the amount of isocyanate applied being in the range of from 20 to 80% of the amount calculated from the number of amino and guanidino groups present, decomposing the cross-linked products in an aqueous solution at a temperature of from 60 to 150 °C up to a molecular weight of from 10.000 to 100.000 and adjusting the pH of the solutions obtained to a value of 7.

**Revendications**

1. Mélange stabilisé, constitué de:
a) un nicotinamide-adénine-dinucléotide sous forme réduite ou oxydée ou un phosphate de nicotinamide-adénine-dinucléotide sous forme réduite ou oxydée, et
b) une gélatine réticulée.

2. Mélange selon la revendication 1, caractérisé par un rapport nucléotide/gélatine réticulée compris entre 1:0,001 et 1:1200.

3. Utilisation de gélatine réticulée comme stabilisant pour une nicotinamide-adénine-dinucléotide sous forme réduite ou oxydée ou pour une phosphate de nicotinamide-adénine-dinucléotide sous forme réduite ou oxydée.

4. Procédé de stabilisation d'un nicotinamide-adénine-dinucléotide sous forme réduite ou oxydée ou d'un phosphate de nicotinamide-adénine-dinucléotide sous forme réduite ou oxydée, caractérisé en ce qu'on traite une solution du nucléotide avec une gélatine réticulée et on sèche si on le désire.

5. Procédé selon la revendication 4, caractérisé en ce qu'on utilise une gélatine réticulée qui est préparée en dégradant du collagène ou un de ses produits de dégradation en solution aqueuse à une température de 60 à 150 °C, jusqu'à une masse moléculaire de 2000 à 20 000, de préférence de 5000 à 10 000, en faisant réagir le collagène dégradé avec un diisocyanate à des températures de 0 à 100 °C, dans une gamme de pH neutre à faiblement alcaline, éventuellement en présence d'un solvant organique inerte de telle manière que la quantité d'isocyanate utilisée soit plus faible que la quantité calculée d'après le nombre de groupes amino et guanidino présents dans le collagène dégradé et atteigne de préférence de 20 à 80% de cette quantité, et en réglant le produit de réticulation formé à une valeur de pH d'environ 7, ou bien en faisant réagir des produits de dégradation du collagène avec des diisocyanates à des températures comprises entre 0 et 100 °C dans une gamme de pH neutre à faiblement alcaline, éventuellement en présence d'un solvant organique inerte, auquel cas la quantité d'isocyanate utilisée atteint de 20 à 80% de celle calculée à partir du nombre de groupes amino et guanidino présents, les produits de réticulation formés sont dégradés en solution aqueuse à des températures comprises entre 60 et 150 °C jusqu'à une masse moléculaire de 10 000 à 100 000 et les solutions obtenues sont réglées à une valeur de pH de 7.